# EUROPEAN PATENT SPECIFICATION

(11) **EP 4 210 609 B1**
(45) Date of publication and mention of the grant of the patent: **22.07.2026**
(21) Application number: 20953402.3
(22) Date of filing: 14.09.2020
(51) Int. Cl.: A61B 18/14, A61B 17/00, A61B 18/00

(54) **SMOKE PENCIL WITH SWIVEL DEVICE**
RAUCHSTIFT MIT SCHWENKVORRICHTUNG
CRAYON À FUMÉE DOTÉ D'UN DISPOSITIF PIVOTANT

(43) Date of publication of application: 19.07.2023
(73) Proprietor: BIO-PROTECH INC., Gangwon-do, 26365 (KR)
(72) Inventor: PARK, Ik Ro, Wonju-si Gangwon-do 26365 (KR)
(74) Representative: Verscht, Thomas Kurt Albert
(86) International application number: PCT/KR2020/012376
(87) International publication number: WO 2022/055000

(56) References cited:
- EP-B1- 2 890 322
- US-A1- 2009 018 539
- US-A1- 2009 018 539
- US-A1- 2009 062 791
- US-A1- 2009 062 791
- US-A1- 2011 190 768
- US-A1- 2018 036 064
- US-A1- 2020 093 535

## Description

### Technical Field

The present invention relates to a smoke pencil with swivel device and, more particularly, to a smoke pencil with swivel device that guides a cable and a suction hose downward when a user rotates a body of the smoke pencil with swivel device.

### Background Art

In general, a smoke pencil is representative medical equipment that is used to cut a portion of a tissue of a human body or coagulate a tissue or blood using electricity instead of scalpels usually in surgical operations.

Such a smoke pencil, which uses a principle that a high-frequency current generates a short spark or heat without electrically shocking or stimulating on muscles when flowing through a human body, cuts a desired tissue using high-frequency energy of about 100°C or coagulates a desired tissue using high-frequency energy of about 60°C.

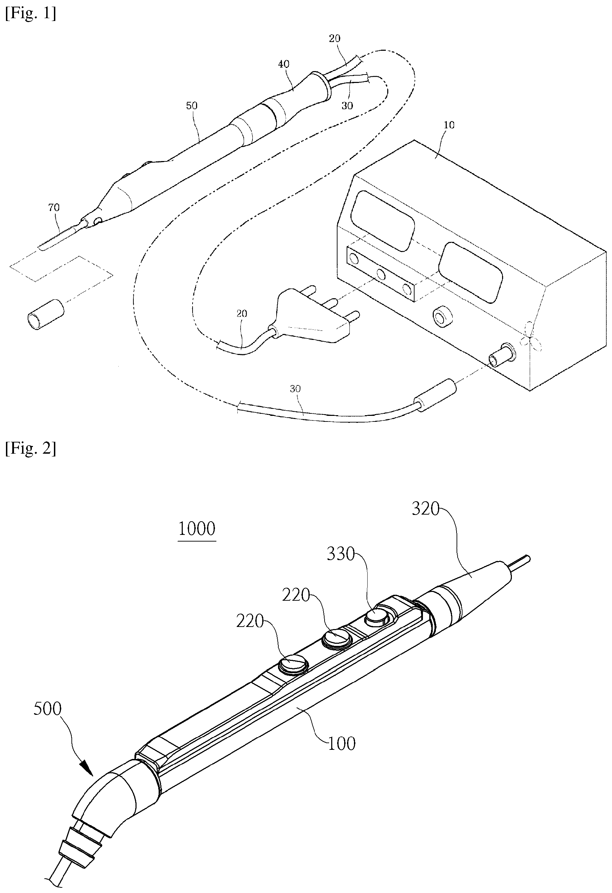

FIG 1 is a view showing a smoke pencil of the related art. Referring to FIG 1, a smoke pencil of the related art, which has been disclosed in Korean Patent Application Publication No. 10-2001-0035322, includes a suction cannula 50, a scalpel 70 coupled to an end of the suction cannula 50 to be heated by high-frequency energy, and a supporting tube 40 coupled to another end of the suction cannula 50. A cable 20 and a suction hose 30 are exposed out of the suction cannula 50 through the supporting tube 40. The cable 20 and the suction hose 30 exposed in this way are connected to an external device 10. A odor suctioned through the suction cannula 50 is discharged to the external device 10 through the suction cannula 50, and the scalpel 70 receiving high-frequency energy from the external device 10 connected with the cable 20 is used for cutting or homestasis.

Meanwhile, a user rotates and uses a smoke pencil at various angles while performing an operation using the smoke pencil, depending on the shapes of affected parts. Since the external device 10 is fixed at one side of the operating room even if the smoke pencil is rotated, the cable 20 and the suction hose 30 exposed out of the supporting tube 40 have to face the external device 10. However, the smoke pencil has no specific configuration that guides the cable 20 and the suction hose 30 exposed out of the supporting tube 40 toward the external device 10, so there is a problem that a user has difficulty in rotating the smoke pencil.

With regard to the prior art attention is drawn to US 2020/093535 A1 from which an electrosurgical smoke evacuation pencil is known, which includes a handle housing having a proximal end portion and a distal end portion, the handle housing defining a first lumen therethrough. The electrosurgical pencil also includes a nozzle disposed within the first lumen and defining a second lumen, the nozzle being movable relative to and within the handle housing and extending proximally past the proximal end portion of the handle housing. The electrosurgical pencil may further include a swivel connector coupled to the distal end portion of the handle housing, the swivel connector configured to couple to a suction source. The electrosurgical pencil includes a hub assembly securedly disposed within the second lumen, the hub assembly including a conductive tube configured to couple to a source of electrosurgical energy, and an electrode slidably disposed within the conductive tube, the electrode being movable relative to and within the conductive tube.

### Disclosure of Invention

### Solution to Problem

The present invention has been made in an effort to solve the problems and an objective of the present invention is to provide a smoke pencil with swivel device that enables a user to easily rotate a body of the smoke pencil with swivel device by guiding a cable and suction hose downward when the user rotates the body.

In order to achieve the objectives of the present invention, a smoke pencil with swivel device, for cutting a portion of a tissue of a human body or for coagulating a tissue or blood, wherein the smoke pencil with swivel device includes: a body longitudinally elongated, having a space longitudinally formed therein, having a blade disposed at a longitudinal front of the space to be exposed outside, and having a coupling portion protruding from a longitudinal rear of the space; an operation part including a substrate disposed at a side of the space and electrically connected with the blade, an operator connected to the substrate and exposed over the space, and a cable having an end electrically connected to the substrate and another end extending out of the body through the space and the coupling portion; and a rotation guide including a first case rotatably disposed on a side of an outer surface of the coupling portion, a second case rotatably disposed on another side of the outer surface of the coupling portion and combined with the first case, an inflow air cannula sealing a joint between a side of the coupling portion and first case and a joint between another side of the coupling portion and the second case.

A first end portion of the first case may cover a side of the outer surface of the coupling portion and a second end portion of the first case may be bent downward; and a first end portion of the second case may cover another side of the outer surface of the coupling portion and a second end portion of the second case may be bent downward.

A rotation guide protrusion may be formed on the outer surface of the coupling portion; a first rotation guide slit may be formed on an inner surface of the first case so that a side of the rotation guide protrusion is inserted therein, and a second rotation guide slit may be formed on an inner surface of the second case so that another side of the rotation guide protrusion is inserted therein; and when the body is rotated, the rotation guide protrusion may be rotated in the first rotation guide slit and the second rotation guide slit.

An outer surface of the rotation guide protrusion may slide in contact with an inner surface of the first rotation guide slit and an inner surface of the second rotation guide slit.

The rotation guide protrusion may protrude from an outer surface of a longitudinal end portion of the coupling portion and a rotation support protrusion may protrude from an outer surface of another longitudinal end portion; the first rotation guide slit may be formed an inner surface of a longitudinal portion of the first case and a first rotation support slit may be formed on an inner surface of another longitudinal portion of the first case so that a side of the rotation support protrusion is inserted therein; and the second rotation guide slit may be formed an inner surface of a longitudinal portion of the second case and a second rotation support slit may be formed on an inner surface of another longitudinal portion of the second case so that another side of the rotation support protrusion is inserted therein.

A plurality of shaved portions may be formed inward from a predetermined circumference of the rotation support protrusion on an outer surface of the rotation support protrusion, and when the outer surface of the rotation support protrusion is positioned to come in contact with an inner surface of the first rotation support slit and an inner surface of the second rotation support slit, the shaved portions may be spaced part from the inner surface of the first rotation support slit and the inner surface of the second rotation support slit.

The inflow air cannula tube may have an insertion portion formed in a rod shape to be inserted into the coupling portion, and a bending portion bending toward the first case and the second case at a rear end of the insertion portion positioned outside the coupling portion.

A first guide groove may be formed on an inner surface of the first case so that a side of an outer surface of the bending portion is inserted therein, a second guide groove may be formed on an inner surface of the second case so that another side of the outer surface of the bending portion is inserted therein, and the outer surface of the bending portion may be in contact with an inner surface of the first guide groove and an inner surface of the second guide groove.

A contact portion may protrude from a front end of the insertion portion positioned in the coupling portion to be in contact with an inner surface of the coupling portion, and an outer surface of the insertion portion inserted in the coupling portion may be spaced apart from the inner surface of the coupling portion.

The contact portion may be tapered as the contact portion goes away from the insertion portion.

An inclined portion protruding away from a rear end of the insertion portion may be formed at the rear end of the insertion portion, the bending portion may be formed at an end of the inclined portion that is farthest from the rear end of the insertion portion, the inclined portion may increase in diameter as the inclined portion goes away from the rear end of the insertion portion, and the inclined portion may not be inserted in the first guide groove and the second guide groove so that the rear end of the insertion portion and the first and second grooves are spaced apart from each other.

A first end portion of the first case and a first end portion of the second case may be coupled to the coupling portion, a second end portion of the first case and a second end portion of the second case may be inclined downward, a first wire guide protrusion may be formed on an inner surface between the first end portion and the second end portion of the first case, a second wire guide protrusion may be formed on an inner surface between the first end portion and the second end portion of the second case to be in contact with the first wire guide protrusion, and when the cable is disposed between the first case and the second case, the cable may be placed over the first wire guide protrusion and the second wire guide protrusion and guided out of the first case and the second case.

### Advantageous Effects of Invention

According to the present invention, since the first end portions of the first and second cases are rotatably coupled to a body and second end portions of the first case and the second case are inclined downward, when a user rotates the body, a cable and a suction hose are guided downward. Accordingly, there is an effect that a user can easily rotate the body.

Further, when the body is rotated, the rotation guide protrusion and the rotation support protrusion are rotated in the first and second rotation guide slits and the first and second rotation support slits, so there is an effect that the coupling portion is stably rotated while being firmly supported inside the first and second cases.

Further, since external air cannot flow into the space defined by the first and second cases, the space defined by the first and second cases is maintained at a predetermined negative pressure, so there is an effect that the smoke moving toward the first and second cases through the coupling portion is easily discharged outside from the first and second cases.

Further, since the rotation support protrusion has the shaved portions, the friction between the rotation support protrusion and the first and second rotation support slits decreases, so there is an effect that the rotation support protrusion is easily inserted while being guided by the first and second rotation support slits.

Further, since the bending portion is fitted in the first and second guide grooves, the smoke flowing to the space defined by the first and second cases through the coupling portion is prevented from leaking outside through between the bending portion and the first and second guide grooves, and air outside the first and second guide grooves is prevented from flowing into the space defined by the first and second cases through between the bending portion and the first and second guide grooves.

Further, since the insertion portion has the contact portion and the inclined portion so that the outer surface of the insertion portion and the inner surface of the coupling portion are spaced apart from each other, there is no friction between the insertion portion and the coupling portion, so there is an effect that the coupling portion is easily rotated.

Further, since the cable guided in the space defined by the first and second cases is placed over the first and second wire guide protrusions, there is no friction between the cable and the inner surfaces of the first and second cases, so there is an effect that the cable is easily moved in the space defined by the first and second cases.

### Brief Description of Drawings

The above and other objects, features and other advantages of the present invention will be more clearly understood from the following detailed description when taken in conjunction with the accompanying drawings, in which:
FIG. 1 is a view showing a smoke pencil of the related art;
FIG. 2 is a view schematically showing a smoke pencil with swivel device according to an exemplary embodiment of the present invention;
FIG. 3 is a view schematically showing a cross-section of the smoke pencil with swivel device according to an exemplary embodiment of the present invention;
FIG. 4 is an exploded view showing a rotation guide of the smoke pencil with swivel device according to an exemplary embodiment of the present invention seen from a side;
FIG. 5 is an exploded view showing the rotation guide of the smoke pencil with swivel device according to an exemplary embodiment of the present invention seen from another side;
FIG. 6 is a view showing a combination of the rotation guide and a body of the smoke pencil with swivel device according to an exemplary embodiment of the present invention;
FIG. 7 is a cross-sectional view taken along line A-A' of FIG. 3; and
FIG. 8 is a cross-sectional view showing a combination of the rotation guide and a body of the smoke pencil with swivel device according to an exemplary embodiment of the present invention.

### Mode for the Invention

Hereinafter, a smoke pencil with swivel device according to an exemplary embodiment of the present invention is described in detail with reference to the accompanying drawings.

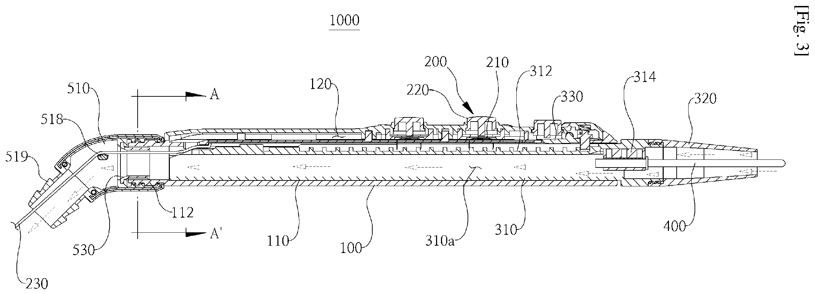

FIG. 2 is a view schematically showing a smoke pencil with swivel device according to an exemplary embodiment of the present invention and FIG. 3 is a view schematically showing a cross-section of the smoke pencil with swivel device according to an exemplary embodiment of the present invention.

Referring to FIGS. 2 and 3, a smoke pencil with swivel device 1000 having a rotation guide according to an exemplary embodiment of the present invention, which is used for a user such as a doctor to cut a portion of a tissue of the patient's body or coagulate blood, includes a body 100, an operation part 200, a stretcher 300, a blade 400, and a rotation guide 500.

Hereafter, unless specifically stated, when a user holds the body 100 with a hand and cuts or coagulates a tissue of a patient's body using the blade 400, the side close to the tissue is referred to as 'forward' or 'front' and the opposite side is referred to as 'rearward' or 'rear'.

The body 100 is a part that a user holds with a hand, is longitudinally elongated, and has a lower space 110 and an upper space 120 positioned over the lower space 110. The lower space 110 and the upper space 120 are separated as independent spaces. The lower space 110 suctions smoke produced when a tissue of a patient's body is cut or coagulated by the blade 400, and the suctioned smoke flows in the lower space 110. The blade 400 is exposed outside through the longitudinal front of the lower space 110 and a coupling portion 112 protrudes from the longitudinal rear of the lower space 110. The upper space 120 provides a space in which a substrate 210 to be described below is disposed.

The operation part 200 includes the substrate 210, an operator 220, and a cable 230. The substrate 210 may be, for example, a Printed Circuit Board (PCB), is disposed in the upper space 120, is electrically connected with the blade 400, and transmits high-frequency energy to the blade 400. The operator 220, for example, may be one or more buttons or touch panels and has a bottom connected to the substrate 210 and a top exposed over the upper space 120. When a user operates the top of the operator 220 exposed upward from the upper space 120, the substrate 210 adjusts the amount of high-frequency energy received from the outside and then transmits the high-frequency energy to the blade 400. The cable 230 may be a wire, etc., and has an end electrically connected to the rear of the substrate 210 and another end extending out of the body 100 through the rear of the lower space 110 and the coupling portion 112. The cable 230 extending out of the body 100 is connected to an external high-frequency generator (not shown) and transmits high-frequency energy generated by the high-frequency generator to the substrate 210.

The stretcher 300 includes a slider 310, an extension 320, a button stopper 330, and a contact guide (not shown). The slider 310 has a long shape to form a suction channel 310a through which smoke is suctioned, and can slide in the lower space 110. The slider 310 has locking steps 312 and a coupling guide 314. The locking steps 312 are recessed and longitudinally arranged on the top of the slider 310. The coupling guide 314 is disposed at the front of the suction channel 310a in the slider 310 and is made of a conductive material, and the blade 400 is coupled to the coupling guide 314. The extension 320 is coupled to the front of the slider 310. The button stopper 330 is supported at the front portion of the upper space 120 and is moved up and down to be locked to or unlocked from the locking steps 312. The contact guide has a side electrically connected to the substrate 210 and another side electrically connected to the coupling guide 314, thereby electrically connecting substrate 210 and the blade 400 to each other. When the button stopper 330 is positioned to be unlocked from the locking step 312 by a user, the slider 310 can slide through the lower space 110, and when the button stopper 330 is locked to the locking step 312, the slider 310 is fixed in the lower space 110. It is possible to adjust the length of the entire body 100 by controlling the length of the slider 310 protruding forward from the lower space 110 using the button stopper 330. The stretcher 300 may be omitted, if necessary. In this case, the coupling guide 314 may be disposed at the front of the lower space 110.

The blade 400 is longitudinally elongated and has an end coupled to the coupling guide 314 and electrically connected with the substrate 210 and another end extending forward from the suction channel 310a to be exposed forward out of the extension 320. When high-frequency energy is transmitted from the substrate 210 to the blade 400, the blade 400 cuts or coagulates a portion of a tissue of a patient's body using the high-frequency energy. When the blade 400 cuts or coagulates a portion of a tissue of a human body, smoke spreading around the blade 400 is suctioned into the suction channel 310a and then discharged outside through the rotation guide 500.

The rotation guide 500 is rotatably coupled to the coupling portion 112. An external suction hose (not shown) is connected to a connection tube 519 of the rotation guide 500. The suction hose is connected to a suction device such as a smoke evacuator that suctions air, so the smoke spreading around the blade 400 in an operation is suctioned into the suction channel 310a and then suctioned into the suction device through the rotation guide 500 and the suction hose.

Since the rotation guide 500 is connected to the suction hose, when a user performs an operation while rotating the body 100 at several angles in accordance with the shape of an affected part, the body 100 should be easily rotated while supported by the rotation guide 500. Further, since the smoke suctioned in the body 100 moves to the rotation guide 500 and is then suctioned into the suction device, the smoke moving to the rotation guide 500 through the body 100 should not leak out of the rotation guide 500. The detailed structure of the rotation guide 500 is described in priority of this configuration.

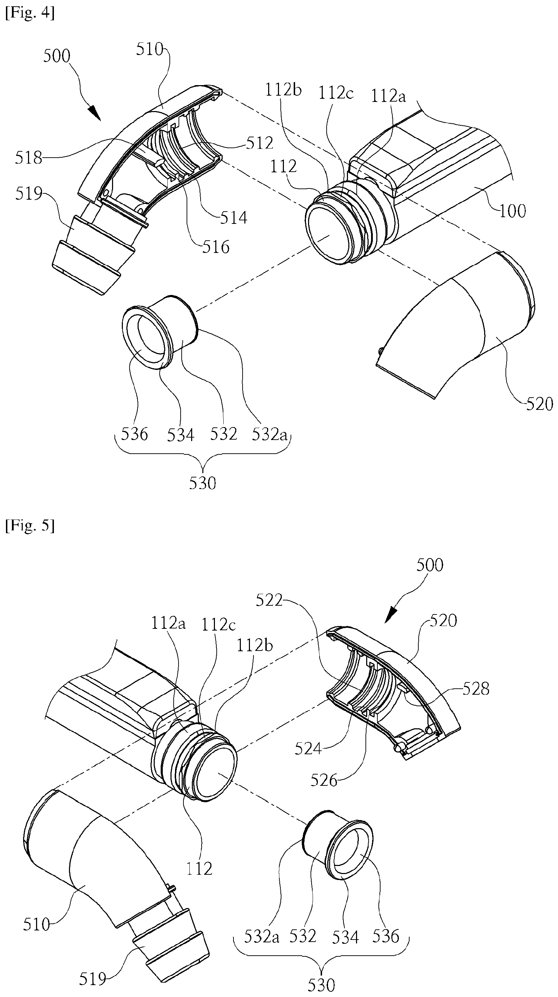

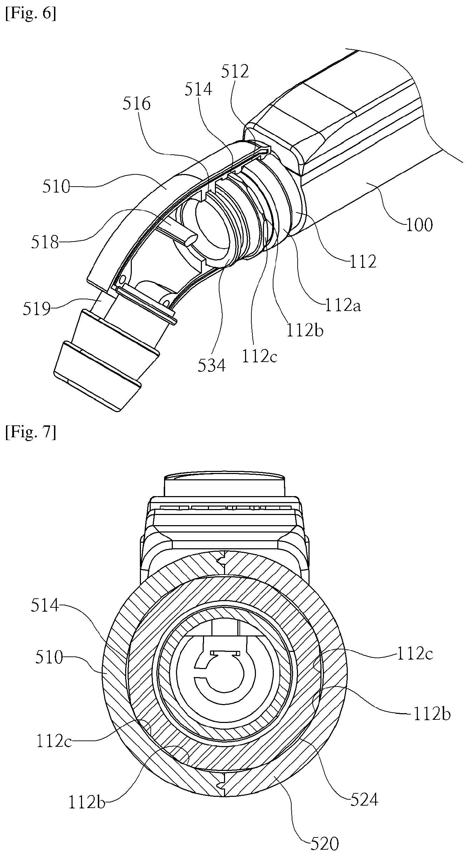

FIG. 4 is an exploded view showing a rotation guide of the smoke pencil with swivel device according to an exemplary embodiment of the present invention seen from a side, FIG. 5 is an exploded view showing the rotation guide of the smoke pencil with swivel device according to an exemplary embodiment of the present invention seen from another side, and FIG. 6 is a view showing a combination of the rotation guide and a body of the smoke pencil with swivel device according to an exemplary embodiment of the present invention.

Referring to FIGS. 4 to 6, the coupling portion 112 having a cylindrical shape integrally protrudes from the rear of the body 100 and the inside of the coupling portion 112 communicates with the lower space 110. A rotation guide protrusion 112a protrudes in a ring shape from the outer surface of the longitudinal front portion of the coupling portion 112 and a rotation support protrusion 112b protrudes in a ring shape from the outer surface of the longitudinal rear portion of the coupling portion 112.

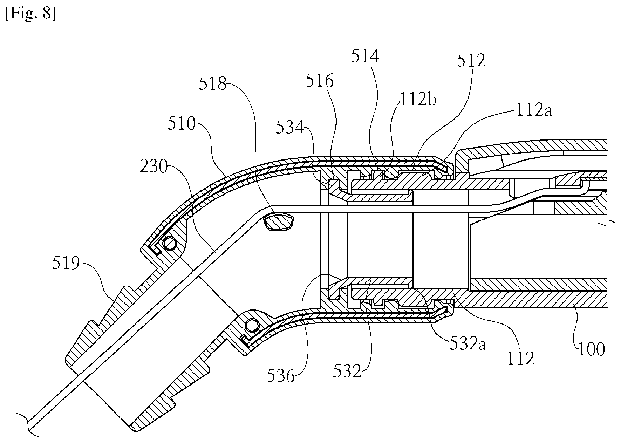

The rotation guide 500 includes a first case 510, a second case 520, and an inflow air cannula 530. The inflow air cannula 530 will be described with reference to FIG. 8.

The first case 510 is provided to cover a side of the outer surface of the coupling portion 112. The second case 520 is provided to cover another side of the outer surface of the coupling portion 112. The sides facing each other of the first case 510 and the second case 520 are fitted to each other. In order to guide coupling to the coupling portion 112, a first rotation guide slit 512 is formed on the inner surface of the longitudinal front portion of the first case 510 so that a side of the rotation guide protrusion 112a is inserted therein, and a first rotation support slit 514 is formed on the inner surface of the longitudinal rear portion of the first case 510 so that a side of the rotation support protrusion 112b is inserted therein. A second rotation guide slit 522 is formed on the outer side of the longitudinal front portion of the second case 520 so that another side of the rotation guide protrusion 112a is inserted therein, and a second rotation support slit 524 is formed on the inner surface of the longitudinal rear portion of the second case 520 so that another side of the rotation support protrusion 112b is inserted therein. Since the coupling portion 112 has the rotation guide protrusion 112a and the rotation support protrusion 112b, when the body 100 is rotated, the rotation guide protrusion 112a and the rotation support protrusion 112b are rotated in the first and second rotation guide slits 512 and 522 and the first and second rotation support slits 514 and 524, so there is an effect that the coupling portion 112 is stably rotated while being firmly supported inside the first and second cases 510 and 520.

Further, since first end portions of the first and second cases 510 and 520 are rotatably coupled to the body 100 and second end portions of the first and second cases 510 and 520 are inclined downward, when a user rotates the body 100, the cable 230 and the suction hose are guided downward through the second end portions of the first and second cases 510 and 520. Accordingly, there is an effect that the user can easily rotate the body 100.

When the first and second cases 510 and 520 are coupled to the coupling portion 112, the outer surface of the rotation guide protrusion 112a comes in contact with the inner surfaces of the first rotation guide slit 512 and the second rotation guide slit 522. Accordingly, when the body 100 is rotated, the rotation guide protrusion 112a slides and rotates in contact with the inner surfaces of the first rotation guide slit 512 and the second rotation guide slit 522. Therefore, there is an effect that the rotation guide protrusion 112a is stably rotated while being guided by the inner surfaces of the first rotation guide slit 512 and the second rotation guide slit 522.

Further, since the outer surface of the rotation guide protrusion 112a is in contact with the inner surfaces of the first and second rotation guide slits 512 and 522, external air cannot come into the spaced defined by the first and second cases 510 and 520 through the space between the first and second rotation guide slits 512 and 522, so the inside defined by the first and second cases 510 and 520 is maintained at a predetermined negative pressure. Accordingly, there is an effect that the smoke moving toward the first and second cases 510 and 520 through the coupling portion 112 is easily discharged outside from the first and second cases 510 and 520.

FIG. 7 is a cross-sectional view taken along line A-A' of FIG. 3.

Referring to FIGS. 6 and 7, a plurality of shaved portions 112c is formed inward from a predetermined circumference of the rotation support protrusion 112b on the outer surface of the rotation support protrusion 112b. The shaved portions 112c are positioned inward from the predetermined circumference of the rotation support protrusion 112b. Accordingly, when the outer surface of the rotation support protrusion 112b is rotated on the inner surfaces of the first and second rotation support slits 514 and 524 by rotation of the coupling portion 112 after the rotation support protrusion 112b is inserted in the first and second rotation support slits 514 and 524, the shaved portions 112c do not come in contact with the inner surfaces of the first and second rotation support slits 514 and 524. Accordingly, the contact area between the outer surface of the rotation support protrusion 112b and the inner surfaces of the first and second rotation support slits 514 and 524 decreases, so friction between the rotation support protrusion 112b and the first and second rotation support slits 514 and 524 is reduced. As described above, since friction between the rotation support protrusion 112b and the first and second rotation support slits 514 and 524 is reduced, there is an effect that the rotation support protrusion 112b is more easily rotated while being guided by the first and second rotation support slits 514 and 524.

FIG. 8 is a cross-sectional view showing a combination of the rotation guide and a body of the smoke pencil with swivel device according to an exemplary embodiment of the present invention.

Referring to FIGS. 4 to 8, the inflow air cannula 530 prevents fluid from flowing between a side of the coupling portion 112 and the first case 510 and between another side of the coupling portion 112 and the second case 520. In detail, the inflow air cannula 530 prevents smoke flowing through the coupling portion 112 from leaking outside while the smoke flows through the space defined between the first and second cases 510 and 520, and prevents air outside the first and second cases 510 and 520 from flowing into the space defined by the first and second cases 510. The inflow air cannula 530 has an insertion portion formed in a rod shape to be inserted into the coupling portion 112 and a bending portion 534 bending toward the first case 510 and the second case 520 at the rear end of the insertion portion 532 positioned outside the coupling portion 112. An inclined portion 536 protruding away from the rear end of the insertion portion 532 is formed at the rear end of the insertion portion 532 and the bending portion 534 may be formed at the end of the inclined portion 536 that is farthest from the rear end of the insertion portion 532. The inclined portion 536 increases in diameter as it goes away from the rear end of the insertion portion 532.

A first guide groove 516 is formed on the inner surface of the first case 510 so that a side of the outer surface of the bending portion 534 is inserted therein, and a second guide groove 526 is formed on the inner surface of the second case 520 so that another side of the outer surface of the bending portion 534 is inserted therein. When the first and second cases 510 and 520 are coupled to the coupling portion 112 after the insertion portion 532 is inserted in the coupling portion 112, the bending portion 534 is inserted into the first and second guide grooves 516 and 526, so the outer surface of the bending portion 534 comes in contact with the inner surface of the first guide groove 516 and the inner surface of the second guide groove 526.

As described above, since the bending portion 534 is fitted in the first and second guide grooves 516 and 526, smoke flowing to the space defined by the first and second cases 510 and 520 through the coupling portion 112 is prevented from leaking outside through between the bending portion 534 and the first and second guide grooves 516 and 526 and air outside the first and second guide grooves 516 and 526 is prevented from flowing into the space defined by the first and second cases 510 and 520 through between the bending portion 534 and the first and second guide grooves 516 and 526.

A contact portion 532a may protrude from the front end of the insertion portion 532 positioned in the coupling portion 112. The contact portion 532a spaces the insertion portion 532 apart from the coupling portion 112 to prevent friction between the insertion portion 532 and the coupling portion 112. That is, the larger the contact area between the coupling portion 112 and the insertion portion 532 inserted in the coupling portion 112, the larger the friction between the insertion portion 532 and the coupling portion 112. Further, the larger the friction between the insertion portion 532 and the coupling portion 112, the more the rotation of the coupling portion 112 is limited. The protrusive contact portion 532a is formed at the front end of the insertion portion 532 to solve this problem in the present invention. Since the outer surface of the insertion portion 532 and the inner surface of the coupling portion 112 are spaced apart from each other by the contact portion 532a, there is no friction between the insertion portion 532 and the coupling portion 112, so there is an effect that the coupling portion 112 is easily rotated. The contact portion 532 may be tapered as it goes away from the insertion portion 532 to minimize the contact area between the contact portion 532a and the coupling portion 112.

The inclined portion 536 is not inserted in the first guide groove 516 and the second guide groove 526 so that the rear end of the insertion portion 532 and the first and second guide grooves 516 and 526 are spaced part from each other. The inclined portion 536 has a similar function to the contact portion 532a, so the rear end of the insertion portion 532 and the first and second guide grooves 516 and 526 are spaced part from each other by the inclined portion 536. Accordingly, there is an effect that there is no friction between the insertion portion 532 and the first and second guide grooves 516 and 526, and the coupling portion 112 is more easily rotated.

Meanwhile, the smoke moving from the coupling portion 112 to the rotation guide 500 is suctioned into the suction device (not shown) through the suction hose (not shown). To this end, for example, the connection tube 519 protrudes from an end of the first case 510 and the suction hose is connected to the connection tube 519. The cable 230 guided inside the first and second cases 510 and 520 from the coupling portion 112 is guided out of the first and second case 510 and 520, that is, toward the suction hose through the connection tube 519. Since the suction hose is usually elongated downward due to gravity, the first and second cases 510 and 520 are bent downward to be easily coupled to the suction hose. In detail, the front portion of the first case 510 covers a side of the outer surface of the coupling portion 112 and the rear portion of the first case 510 is smoothly curved in a predetermined direction. Similarly, the front portion of the second case 520 covers another side of the outer surface of the coupling portion 112 and the rear portion of the second case 520 is smoothly curved in a predetermined direction.

A first wire guide protrusion 518 is formed at the center portion on the inner surface between an end and another end of the first case 510. Similarly, a second wire guide protrusion 528 that is in contact with the first wire guide protrusion 518 is formed at the center portion on the inner surface between an end and another end of the second case 520. When the cable 230 is disposed between the first case 510 and the second case 520, the cable 230 is placed over the first wire guide protrusion 518 and the second wire guide protrusion 528 and guided out of the first case 510 and the second case 520. When the cable 230 is guided inside the first and second case 510 and 520 and placed over the first and second wire guide protrusions 518 and 528, the cable 230 is spaced apart from the inner surfaces of the first and second cases 510 and 520. Accordingly, there is no friction between the cable 230 and the inner surfaces of the first and second cases 510 and 520, so when the body 100 is rotated and the cable 230 is correspondingly moved in the space defined by the first and second cases 510 and 520. Therefore, there is an effect that the cable 230 is easily moved in the space defined by the first and second cases 510 and 520.

Although the present invention was described above with reference to the embodiment, the present invention is not limited to the embodiment and it is apparent to those skilled in the art that the present invention may be changed and modified in various ways within the scope of the present invention. Further, the changes and modifications should be construed as being included in the present invention if they belong to the claims.

## Claims

1. A smoke pencil with swivel device (1000), for cutting a portion of a tissue of a human body or for coagulating a tissue or blood, the smoke pencil with swivel device (1000) comprising:
a body (100) longitudinally elongated, having a space longitudinally formed therein, having a blade (400) disposed at a longitudinal front of the space to be exposed outside, and having a coupling portion (112) protruding from a longitudinal rear of the space;
an operation part (200) including a substrate (210) disposed at a side of the space and electrically connected with the blade (400), an operator (220) connected to the substrate (210) and exposed over the space, and a cable (230) having an end electrically connected to the substrate (210) and another end extending out of the body (100) through the space and the coupling portion (112); and
a rotation guide (500) including a first case (510) rotatably disposed on a side of an outer surface of the coupling portion (112), a second case (520) rotatably disposed on another side of the outer surface of the coupling portion (112) and combined with the first case (510), and an inflow air cannula (530) sealing a joint between a side of the coupling portion (112) and first case (510) and a joint between another side of the coupling portion (112) and the second case (520).

2. The smoke pencil with swivel device (1000) of claim 1, wherein a first end portion of the first case (510) covers a side of the outer surface of the coupling portion (112) and a second end portion of the first case (510) is bent downward, and
a first end portion of the second case (520) covers another side of the outer surface of the coupling portion (112) and a second end portion of the second case (520) is bent downward.

3. The smoke pencil with swivel device (1000) of claim 1, wherein a rotation guide protrusion (112a) is formed on the outer surface of the coupling portion (112);
a first rotation guide slit (512) is formed on an inner surface of the first case (510) so that a side of the rotation guide protrusion (112a) is inserted therein, and a second rotation guide slit (522) is formed on an inner surface of the second case (520) so that another side of the rotation guide protrusion (112a) is inserted therein; and
when the body (100) is rotated, the rotation guide protrusion (112a) is rotated in the first rotation guide slit (512) and the second rotation guide slit (522).

4. The smoke pencil with swivel device (1000) of claim 3, wherein an outer surface of the rotation guide protrusion (112a) can slide in contact with an inner surface of the first rotation guide slit (512) and an inner surface of the second rotation guide slit (522).

5. The smoke pencil with swivel device (1000) of claim 4, wherein the rotation guide protrusion (112a) protrudes from an outer surface of a longitudinal end portion of the coupling portion (112) and a rotation support protrusion (112b) protrudes from an outer surface of another longitudinal end portion;
the first rotation guide slit (512) is formed on an inner surface of a longitudinal portion of the first case (510) and a first rotation support slit (514) is formed on an inner surface of another longitudinal portion of the first case (510) so that a side of the rotation support protrusion (112b) is inserted therein; and
the second rotation guide slit (522) is formed on an inner surface of a longitudinal portion of the second case (520) and a second rotation support slit (524) is formed on an inner surface of another longitudinal portion of the second case (520) so that another side of the rotation support protrusion (112b) is inserted therein.

6. The smoke pencil with swivel device (1000) of claim 5, wherein a plurality of shaved portions (112c) is formed inward from a predetermined circumference of the rotation support protrusion (112b) on an outer surface of the rotation support protrusion (112b), and
when the outer surface of the rotation support protrusion (112b) is positioned to come in contact with an inner surface of the first rotation support slit (514) and an inner surface of the second rotation support slit (524), the shaved portions (112c) are spaced apart from the inner surface of the first rotation support slit (514) and the inner surface of the second rotation support slit (524).

7. The smoke pencil with swivel device (1000) of claim 1, wherein the inflow air cannula (530) has an insertion portion (532) formed in a rod shape to be inserted into the coupling portion (112), and a bending portion (534) bending toward the first case (510) and the second case (520) at a rear end of the insertion portion (532) positioned outside the coupling portion (112).

8. The smoke pencil with swivel device (1000) of claim 7, wherein a first guide groove (516) is formed on an inner surface of the first case (510) so that a side of an outer surface of the bending portion (534) is inserted therein,
a second guide groove (526) is formed on an inner surface of the second case (520) so that another side of the outer surface of the bending portion (534) is inserted therein, and
the outer surface of the bending portion (534) is in contact with an inner surface of the first guide groove (516) and an inner surface of the second guide groove (526).

9. The smoke pencil with swivel device (1000) of claim 8, wherein a contact portion (532a) protrudes from a front end of the insertion portion (532) positioned in the coupling portion (112) to be in contact with an inner surface of the coupling portion (112), and
an outer surface of the insertion portion (532) inserted in the coupling portion (112) is spaced apart from the inner surface of the coupling portion (112).

10. The smoke pencil with swivel device (1000) of claim 9, wherein the contact portion (532a) is tapered as the contact portion (532a) goes away from the insertion portion (532).

11. The smoke pencil with swivel device (1000) of claim 8, wherein an inclined portion (536) protruding away from a rear end of the insertion portion (532) is formed at the rear end of the insertion portion (532),
the bending portion (534) is formed at an end of the inclined portion (536) that is farthest from the rear end of the insertion portion (532),
the inclined portion (536) increases in diameter as the inclined portion (536) goes away from the rear end of the insertion portion (532), and
the inclined portion (536) is not inserted in the first guide groove (516) and the second guide groove (526) so that the rear end of the insertion portion (532) and the first and second grooves (516, 526) are spaced apart from each other.

12. The smoke pencil with swivel device (1000) of claim 1, wherein a first end portion of the first case (510) and a first end portion of the second case (520) are coupled to the coupling portion (112),
a second end portion of the first case (510) and a second end portion of the second case (520) are inclined downward,
a first wire guide protrusion (518) is formed on an inner surface between the first end portion and the second end portion of the first case (510),
a second wire guide protrusion (528) is formed on an inner surface between the first end portion and the second end portion of the second case (520) to be in contact with the first wire guide protrusion (518), and when the cable (230) is disposed between the first case (510) and the second case (520), the cable (230) is placed over the first wire guide protrusion (518) and the second wire guide protrusion (528) and guided out of the first case (510) and the second case (520).

## Patentansprüche

1. Ein Rauchstift mit Schwenkvorrichtung (1000), zum Schneiden eines Teils eines Gewebes eines menschlichen Körpers oder zum Koagulieren von Gewebe oder Blut, wobei der Rauchstift mit Schwenkvorrichtung (1000) folgendes aufweist:
einen in Längsrichtung langgestreckten Körper (100), mit einem Raum, der in Längsrichtung darin gebildet ist, mit einer Klinge (400), die an einer Längsvorderseite des Raums angeordnet ist, um nach außen freigelegt zu sein, und mit einem Kupplungsteil (112), der von einer Längsrückseite des Raums vorsteht;
ein Bedienungsteil (200), das ein Substrat (210), welches an einer Seite des Raums angeordnet ist und elektrisch mit der Klinge (400) verbunden ist, eine Bedienungseinrichtung (220), die mit dem Substrat (210) verbunden ist und über dem Raum freiliegt, und ein Kabel (230), mit einem Ende, das elektrisch mit dem Substrat (210) verbunden ist, und einem weiteren Ende, das aus dem Körper (100) durch den Raum und den Kupplungsteil (112) vorsteht, aufweist; und
eine Drehführung (500), die ein erstes Gehäuse (510), das drehbar auf einer Seite einer Außenoberoberfläche des Kupplungsteils (112) angeordnet ist, ein zweites Gehäuse (520), das drehbar auf einer weiteren Seite der Außenoberoberfläche des Kupplungsteils (112) angeordnet ist und mit dem ersten Gehäuse (510) kombiniert ist, und eine Einströmluftkanüle (530), die eine Verbindung zwischen einer Seite des Kupplungsteils (112) und dem ersten Gehäuse (510) und eine Verbindung zwischen einer weiteren Seite des Kupplungsteils (112) und dem zweiten Gehäuse (520) abdichtet, aufweist.

2. Der Rauchstift mit Schwenkvorrichtung (1000) nach Anspruch 1, wobei ein erster Endteil des ersten Gehäuses (510) eine Seite der Außenoberoberfläche des Kupplungsteils (112) abdeckt, und ein zweiter Endteil des ersten Gehäuses (510) ist nach unten gebogen, und
ein erster Endteil des zweiten Gehäuses (520) deckt eine weitere Seite der Außenoberfläche des Kupplungsteils (112) ab, und ein zweiter Endteil des zweiten Gehäuses (520) ist nach unten gebogen.

3. Der Rauchstift mit Schwenkvorrichtung (1000) nach Anspruch 1, wobei ein Drehführungsvorsprung (112a) auf der Außenoberoberfläche des Kupplungsteils (112) gebildet ist;
ein erster Drehführungsschlitz (512) ist auf einer Innenoberfläche des ersten Gehäuses (510) gebildet, so dass eine Seite des Drehführungsvorsprungs (112a) darin eingesetzt ist, und ein zweiter Drehführungsschlitz (522) ist auf einer Innenoberfläche des zweiten Gehäuses (520) gebildet, so dass eine weitere Seite des Drehführungsvorsprungs (112a) darin eingesetzt ist; und
wenn der Körper (100) gedreht wird, wird der Drehführungsvorsprung (112a) in dem ersten Drehführungsschlitz (512) und dem zweiten Drehführungsschlitz (522) gedreht.

4. Der Rauchstift mit Schwenkvorrichtung (1000) nach Anspruch 3, wobei eine Außenoberfläche des Drehführungsvorsprungs (112a) in Kontakt mit einer Innenoberfläche des ersten Drehführungsschlitzes (512) und einer Innenoberfläche des zweiten Drehführungsschlitzes (522) gleiten kann.

5. Der Rauchstift mit Schwenkvorrichtung (1000) nach Anspruch 4, wobei der Drehführungsvorsprung (112a) von einer Außenoberfläche eines Längsendteils des Kupplungsteils (112) vorsteht, und ein Drehstützvorsprung (112b) von einer Außenoberfläche eines weiteren Längsendteils vorsteht;
der erste Drehführungsschlitz (512) ist auf einer Innenoberfläche eines Längsteils des ersten Gehäuses (510) gebildet, und ein erster Drehstützschlitz (514) ist auf einer Innenoberfläche eines weiteren Längsteils des ersten Gehäuses (510) gebildet, so dass eine Seite des Drehstützvorsprung (112b) darin eingesetzt ist; und
der zweite Drehführungsschlitz (522) ist auf einer Innenoberfläche eines Längsteils des zweiten Gehäuses (520) gebildet, und ein zweiter Drehstützschlitz (524) ist auf einer Innenoberfläche eines weiteren Längsteils des zweiten Gehäuses (520) gebildet, so dass eine weitere Seite des Drehstützvorsprung (112b) darin eingesetzt ist.

6. Der Rauchstift mit Schwenkvorrichtung (1000) nach Anspruch 5, wobei eine Vielzahl von abgeschabten Teilen (112c) von einem vorbestimmten Umfang des Drehstützvorsprungs (112b) nach innen auf einer Außenoberfläche des Drehstützvorsprungs (112b) gebildet ist, und
wenn die Außenoberfläche des Drehstützvorsprungs (112b) positioniert ist, um mit einer Innenoberfläche des ersten Drehstützschlitzes (514) und einer Innenoberfläche des zweiten Drehstützschlitz (524) in Kontakt zu kommen, sind die abgeschabten Teile (112c) von der Innenoberfläche des ersten Drehstützschlitzes (514) und der Innenoberfläche des zweiten Drehstützschlitzes (524) auseinander beabstandet.

7. Der Rauchstift mit Schwenkvorrichtung (1000) nach Anspruch 1, wobei die Einströmluftkanüle (530) einen Einsetzteil (532), der in einer Stabform gebildet ist, um in den Kupplungsteil (112) eingesetzt zu werden, und einen Biegeteil (534), der sich zu dem ersten Gehäuse (510) und dem zweiten Gehäuse (520) an einem hinteren Ende des Einsetzteils (532), das außerhalb des Kupplungsteils (112) positioniert ist, hin biegt, hat.

8. Der Rauchstift mit Schwenkvorrichtung (1000) nach Anspruch 7, wobei eine erste Führungsnut (516) auf einer Innenoberfläche des ersten Gehäuses (510) gebildet ist, so dass eine Seite einer Außenoberfläche des Biegeteils (534) darin eingesetzt ist,
eine zweite Führungsnut (526) ist auf einer Innenoberfläche des zweiten Gehäuses (520) gebildet, so dass eine weitere Seite der Außenoberfläche des Biegeteils (534) darin eingesetzt ist, und
die Außenoberfläche des Biegeteils (534) ist in Kontakt mit einer Innenoberfläche der ersten Führungsnut (516) und einer Innenoberfläche der zweiten Führungsnut (526).

9. Der Rauchstift mit Schwenkvorrichtung (1000) nach Anspruch 8, wobei ein Kontaktteil (532a) von einem vorderen Ende des Einsetzteils (532), das in dem Kupplungsteil (112) positioniert ist, vorsteht, um mit einer Innenoberfläche des Kupplungsteils (112) in Kontakt zu stehen, und
eine Außenoberfläche des Einsetzteils (532), das in dem Kupplungsteil (112) eingesetzt ist, ist von der Innenoberfläche des Kupplungsteils (112) auseinander beabstandet.

10. Der Rauchstift mit Schwenkvorrichtung (1000) nach Anspruch 9, wobei der Kontaktteil (532a) verjüngt ist, wenn der Kontaktteil (532a) von dem Einsetzteil (532) weggeht.

11. Der Rauchstift mit Schwenkvorrichtung (1000) nach Anspruch 8, wobei ein geneigter Teil (536), der von einem hinteren Ende des Einsetzteils (532) wegsteht, an dem hinteren Ende des Einsetzteils (532) gebildet ist,
der Biegeteil (534) ist an einem Ende des geneigten Teils (536), der am weitesten von dem hinteren Ende des Einsetzteils (532) entfernt ist, gebildet,
der geneigte Teil (536) nimmt im Durchmesser zu, wenn der geneigte Teil (536) von dem hinteren Ende des Einsetzteils (532) weggeht, und
der geneigte Teil (536) ist nicht in die erste Führungsnut (516) und zweite Führungsnut (526) eingesetzt, so dass das hintere Ende des Einsetzteils (532) und die ersten und zweiten Nuten (516, 526) voneinander auseinander beabstandet sind.

12. Der Rauchstift mit Schwenkvorrichtung (1000) nach Anspruch 1, wobei ein erster Endteil des ersten Gehäuses (510) und ein erster Endteil des zweiten Gehäuses (520) mit dem Kupplungsteil (112) gekuppelt sind,
ein zweiter Endteil des ersten Gehäuses (510) und ein zweiter Endteil des zweiten Gehäuses (520) sind nach unten geneigt,
ein erster Drahtführungsvorsprung (518) ist auf einer Innenoberfläche zwischen dem ersten Endteil und dem zweiten Endteil des ersten Gehäuses (510) gebildet,
ein zweiter Drahtführungsvorsprung (528) ist auf einer Innenoberfläche zwischen dem ersten Endteil und dem zweiten Endteil des zweiten Gehäuses (520) gebildet, um mit dem ersten Drahtführungsvorsprung (518) in Kontakt zu stehen, und
wenn das Kabel (230) zwischen dem ersten Gehäuse (510) und dem zweiten Gehäuse (520) angeordnet ist, ist das Kabel (230) über den ersten Drahtführungsvorsprung (518) und den zweiten Drahtführungsvorsprung (528) platziert und aus dem ersten Gehäuse (510) und dem zweiten Gehäuse (520) herausgeführt.

## Revendications

1. Cautère électrique avec dispositif pivotant (1000), pour couper une portion d'un tissu d'un corps humain ou pour coaguler un tissu ou du sang, le cautère électrique avec dispositif pivotant (1000) comprenant :
un corps (100) allongé longitudinalement, ayant un espace formé longitudinalement à l'intérieur, ayant une lame (400) disposée au niveau d'un avant longitudinal de l'espace à exposer à l'extérieur, et ayant une portion de couplage (112) faisant saillie depuis un arrière longitudinal de l'espace ;
une partie d'opération (200) comportant un substrat (210) disposé sur un côté de l'espace et connecté électriquement à la lame (400), un opérateur (220) connecté au substrat (210) et exposé au-dessus de l'espace, et un câble (230) ayant une extrémité connectée électriquement au substrat (210) et une autre extrémité s'étendant hors du corps (100) à travers l'espace et la portion de couplage (112) ; et
un guide de rotation (500) comportant un premier boîtier (510) disposé en rotation sur un côté d'une surface extérieure de la portion de couplage (112), un second boîtier (520) disposé en rotation sur un autre côté de la surface extérieure de la portion de couplage (112) et combiné au premier boîtier (510), et une canule d'entrée d'air (530) étanchéifiant un joint entre un côté de la portion de couplage (112) et le premier boîtier (510) et un joint entre un autre côté de la portion de couplage (112) et le second boîtier (520).

2. Cautère électrique avec dispositif pivotant (1000) selon la revendication 1, dans lequel une première portion d'extrémité du premier boîtier (510) recouvre un côté de la surface extérieure de la portion de couplage (112) et une seconde portion d'extrémité du premier boîtier (510) est courbée vers le bas, et
une première portion d'extrémité du second boîtier (520) recouvre un autre côté de la surface extérieure de la portion de couplage (112) et une seconde portion d'extrémité du second boîtier (520) est courbée vers le bas.

3. Cautère électrique avec dispositif pivotant (1000) selon la revendication 1, dans lequel une saillie de guide de rotation (112a) est formée sur la surface extérieure de la portion de couplage (112) ;
une première fente de guide de rotation (512) est formée sur une surface intérieure du premier boîtier (510) de sorte qu'un côté de la saillie de guide de rotation (112a) est inséré à l'intérieur, et une seconde fente de guide de rotation (522) est formée sur une surface intérieure du second boîtier (520) de sorte qu'un autre côté de la saillie de guide de rotation (112a) est inséré à l'intérieur ; et
lorsque le corps (100) est tourné, la saillie de guide de rotation (112a) est tournée dans la première fente de guide de rotation (512) et la seconde fente de guide de rotation (522).

4. Cautère électrique avec dispositif pivotant (1000) selon la revendication 3, dans lequel une surface extérieure de la saillie de guide de rotation (112a) peut glisser en contact avec une surface intérieure de la première fente de guide de rotation (512) et une surface intérieure de la seconde fente de guide de rotation (522).

5. Cautère électrique avec dispositif pivotant (1000) selon la revendication 4, dans lequel la saillie de guide de rotation (112a) fait saillie depuis une surface extérieure d'une portion d'extrémité longitudinale de la portion de couplage (112) et une saillie de support de rotation (112b) fait saillie depuis une surface extérieure d'une autre portion d'extrémité longitudinale ;
la première fente de guide de rotation (512) est formée sur une surface intérieure d'une portion longitudinale du premier boîtier (510) et une première fente de support de rotation (514) est formée sur une surface intérieure d'une autre portion longitudinale du premier boîtier (510) de sorte qu'un côté de la saillie de support de rotation (112b) est inséré à l'intérieur ; et
la seconde fente de guide de rotation (522) est formée sur une surface intérieure d'une portion longitudinale du second boîtier (520) et une seconde fente de support de rotation (524) est formée sur une surface intérieure d'une autre portion longitudinale du second boîtier (520) de sorte qu'un autre côté de la saillie de support de rotation (112b) est inséré à l'intérieur.

6. Cautère électrique avec dispositif pivotant (1000) selon la revendication 5, dans lequel une pluralité de portions rabotées (112c) est formée vers l'intérieur à partir d'une circonférence prédéterminée de la saillie de support de rotation (112b) sur une surface extérieure de la saillie de support de rotation (112b), et
lorsque la surface extérieure de la saillie de support de rotation (112b) est positionnée pour entrer en contact avec une surface intérieure de la première fente de support de rotation (514) et une surface intérieure de la seconde fente de support de rotation (524), les portions rabotées (112c) sont espacées de la surface intérieure de la première fente de support de rotation (514) et de la surface intérieure de la seconde fente de support de rotation (524).

7. Cautère électrique avec dispositif pivotant (1000) selon la revendication 1, dans lequel la canule d'entrée d'air (530) présente une portion d'insertion (532) formée selon une configuration de tige pour être insérée dans la portion de couplage (112), et une portion de courbure (534) se courbant vers le premier boîtier (510) et le second boîtier (520) à une extrémité arrière de la portion d'insertion (532) positionnée à l'extérieur de la portion de couplage (112).

8. Cautère électrique avec dispositif pivotant (1000) selon la revendication 7, dans lequel une première rainure de guidage (516) est formée sur une surface intérieure du premier boîtier (510) de sorte qu'un côté d'une surface extérieure de la portion de courbure (534) est inséré à l'intérieur,
une seconde rainure de guidage (526) est formée sur une surface intérieure du second boîtier (520) de sorte qu'un autre côté de la surface extérieure de la portion de courbure (534) est inséré à l'intérieur, et
la surface extérieure de la portion de courbure (534) est en contact avec une surface intérieure de la première rainure de guidage (516) et une surface intérieure de la seconde rainure de guidage (526).

9. Cautère électrique avec dispositif pivotant (1000) selon la revendication 8, dans lequel une portion de contact (532a) fait saillie depuis une extrémité avant de la portion d'insertion (532) positionnée dans la portion de couplage (112) pour être en contact avec une surface intérieure de la portion de couplage (112), et
une surface extérieure de la portion d'insertion (532) insérée dans la portion de couplage (112) est espacée de la surface intérieure de la portion de couplage (112).

10. Cautère électrique avec dispositif pivotant (1000) selon la revendication 9, dans lequel la portion de contact (532a) est effilée à mesure que la portion de contact (532a) s'éloigne de la portion d'insertion (532).

11. Cautère électrique avec dispositif pivotant (1000) selon la revendication 8, dans lequel une portion inclinée (536) faisant saillie à l'opposé d'une extrémité arrière de la portion d'insertion (532) est formée à l'extrémité arrière de la portion d'insertion (532),
la portion de courbure (534) est formée à une extrémité de la portion inclinée (536) qui est la plus éloignée de l'extrémité arrière de la portion d'insertion (532),
la portion inclinée (536) augmente en termes de diamètre à mesure que la portion inclinée (536) s'éloigne de l'extrémité arrière de la portion d'insertion (532), et
la portion inclinée (536) n'est pas insérée dans la première rainure de guidage (516) et la seconde rainure de guidage (526) de sorte que l'extrémité arrière de la portion d'insertion (532) et les première et seconde rainures (516, 526) sont espacées les unes des autres.

12. Cautère électrique avec dispositif pivotant (1000) selon la revendication 1, dans lequel une première portion d'extrémité du premier boîtier (510) et une première portion d'extrémité du second boîtier (520) sont couplées à la portion de couplage (112),
une seconde portion d'extrémité du premier boîtier (510) et une seconde portion d'extrémité du second boîtier (520) sont inclinées vers le bas,
une première saillie de guide-fil (518) est formée sur une surface intérieure entre la première portion d'extrémité et la seconde portion d'extrémité du premier boîtier (510),
une seconde saillie de guide-fil (528) est formée sur une surface intérieure entre la première portion d'extrémité et la seconde portion d'extrémité du second boîtier (520) pour être en contact avec la première saillie de guide-fil (518), et
lorsque le câble (230) est disposé entre le premier boîtier (510) et le second boîtier (520), le câble (230) est placé sur la première saillie de guide-fil (518) et la seconde saillie de guide-fil (528) et guidé hors du premier boîtier (510) et du second boîtier (520).
